# EUROPEAN PATENT SPECIFICATION

(11) **EP 3 743 028 B1**
(45) Date of publication and mention of the grant of the patent: **01.05.2024**
(21) Application number: 19743416.0
(22) Date of filing: 24.01.2019
(51) Int. Cl.: A61F 13/15

(54) **A REUSABLE INCONTINENCE PAD AND ITS USE IN BRIEFS**
WIEDERVERWENDBARE INKONTINENZEINLAGE UND IHRE VERWENDUNG IN UNTERHOSEN
GARNITURE POUR INCONTINENCE RÉUTILISABLE ET SON UTILISATION DANS UN SLIP

(30) Priority: 24.01.2018 DK PA201870051
(43) Date of publication of application: 02.12.2020
(73) Proprietor: VHB APS, 7400 Herning (DK)
(72) Inventor: BERTELSEN, Villy Hyldgaard, 7400 Herning (DK)
(74) Representative: Patrade A/S
(86) International application number: PCT/DK2019/050030
(87) International publication number: WO 2019/145008

(56) References cited:
- WO-A1-2016/051385
- WO-A1-2016/051385
- US-A- 1 041 420
- US-A- 1 041 420
- US-A- 4 678 465
- US-A- 5 429 627
- US-A- 6 117 323
- Flow Of The Goddess Cloth Pads .: "Cloth Menstrual Pad Sewing Tutorial - Sew Along Turned & Topstitched", Youtube, 24 May 2016 (2016-05-24), page 1 pp., XP054979725, Retrieved from the Internet: URL:https://www.youtube.com/watch?v=hlpD00 8JjzI [retrieved on 2019-09-30]
- Flow Of The Goddess Cloth Pads.: "Cloth Menstrual Pad Sewing Tutorial - Exposed Core", Youtube, 26 May 2016 (2016-05-26), page 1 pp., XP054979726, Retrieved from the Internet: URL:https://www.youtube.com/watch?v=xVLfvQ x-dbQ [retrieved on 2019-09-30]
- Flow Of The Goddess Cloth Pads .: "Cloth Menstrual Pad Sewing Tutorial - Sew Along Turned & Topstitched", YouTube, 24 May 2016 (2016-05-24), XP054979725, Retrieved from the Internet: URL:https://www.youtube.com/watch?v=hlpD00 8JjzI [retrieved on 2019-03-13]
- Flow Of The Goddess Cloth Pads.: "Cloth Menstrual Pad Sewing Tutorial - Exposed Core", YouTube, 26 May 2016 (2016-05-26), XP054979726, Retrieved from the Internet: URL:https://www.youtube.com/watch?v=xVLfvQ x-dbQ [retrieved on 2019-03-13]

## Description

### Field of the Invention

The present invention relates to a washable and reusable incontinence pad and its use in two briefs, which are adapted to be used with such a pad.

### Background of the Invention

Disposable incontinence pads are commonly used in briefs and other types of underwear for collecting liquids in the case of involuntary urination. Such pads are often uncomfortable, expensive and not very environmentally friendly.

Relevant methods are known from Flow Of The Goddess Cloth Pads, Cloth Menstrual. Pad Sewing Tutorial - Sew Along Turned & Topstitched, YouTube (https://www.youtube.com/watch?v=hlpD008JjzI) and Amy Nix, Sewing Cloth Pads 101 - Upcycled Pads, YouTube (https://www.youtube.com/watch?v=oXTimqbdDAo),
which disclose a method for production of cloth menstrual pads comprising the following steps:
- providing an absorbing core with a circumference of the absorbing core, a topside, a backside, and a height of the core, where the topside is oriented towards the user and the backside is oriented away from the user when said menstrual pad is in use;
- providing a mostly liquid-impermeable membrane, which has at least the dimensions of the backside of said absorbing core and plus the height of the core;
- placing the liquid-impermeable membrane on top of the absorbing core with the topside of the absorbing core facing the liquid-impermeable membrane, aligning the circumference of the core and liquid-impermeable membrane;
- sewing together the absorbing core and the liquid-impermeable membrane along a first part of the circumference, leaving a second pan of the circumference of the absorbing core non-sewn defining a hole between the connected absorbing core and the liquid impermeable membrane.
- turning inside-out of the product through the hole, thereby the liquid impermeable membrane will encompass the backside of the absorbing core; and
- sewing the non-sewn part of the liquid impermeable membrane to the absorbing core in the second part of the circumference of the absorbing core.

However, this prior art does disclose a pad that has a liquid impermeable membrane only covering the backside of the pad, and not providing a liquid impermeable barrier along the hight of the pad's absorbing core, thereby not providing means for preventing edge leakage.

From WO2016/051385 and US1041420 there are known incontinence pads, that provide side leakage prevention in the form of a solid liquid-impermeable members. However, such solid or hard liquid-impermeable members cannot be incorporated into a sewn flexible and easily wearable reusable pad.

### Object of the Invention

It is an object of the invention to provide a method for producing a washable and reusable incontinence pad. A further object of the invention is a product produced by said method.

Another object of the invention is the use of a product produced by said method in briefs.

Another object of the invention is the use of a product produced by said method together with a liquid-proof bag.

### Description of the Invention

It is an object of the present invention to provide an incontinence pad, which overcomes the above-mentioned disadvantages of the solutions known in the art.

Furthermore, the present invention relates to a method for producing such an incontinence pad.

The method for production of an incontinence pad may as an example comprise the following steps:
Providing an absorbing core with a circumference of the absorbing core, a topside, a backside, and a height of the core. The topside is oriented towards the user and the backside is oriented away from the user when said incontinence pad is in use.

Providing a liquid-impermeable membrane, which has at least the dimensions of the backside of said absorbing core, plus the height of the core, plus twice the distance of said first sewing to said circumference of the absorbing core.

Placing said liquid-impermeable membrane on top of said absorbing core, with the topside of the core facing said liquid-impermeable membrane, aligning said circumference of the absorbing core and said circumference of the liquid-impermeable membrane.

Sewing together said absorbing core and said liquid-impermeable membrane along a first part of the circumference of the absorbing core, which first part of the circumference of the absorbing core constitutes an essential part of the circumference of the absorbing core, thereby leaving a second part of the circumference of the absorbing core non-sewn, defining a hole between the connected absorbing core and the liquid-impermeable membrane.

Turning the inside out of the product through the hole, thereby the liquid-impermeable membrane will encompass the backside of the absorbing core, the height of the core and inwards over an edge area corresponding to the sewing distance from the circumference of said absorbing core.

Folding the non-sewn part of the liquid-impermeable membrane up along the height of and over the absorbing core's edge region in said second part of the circumference of the absorbing core.

Sewing of the non-sewn part of the liquid-impermeable membrane to the absorbing core in said second part of the circumference of the absorbing core.

The method for production of an incontinence pad may further comprise the step of providing an additional layer of the liquid-impermeable membrane, which is folded over in said second part of the circumference of the absorbing core and sewing the folded over additional layer of the liquid-impermeable membrane across said second part of the circumference of the absorbing core, so that the folded over liquid-impermeable membrane is sewn onto the topside and backside of the absorbing core.

The method for production of an incontinence pad may further comprise the following steps:
Folding the liquid-impermeable membrane into folds in areas located close to corners in the absorbing core, when the circumferences of the absorbing core and the circumference of the liquid-impermeable membrane are brought into alignment.

Sewing of said folds.

The method for production of an incontinence pad may further comprise the step of providing said liquid-impermeable membrane with at least two water-proof press-studs.

The method for production of an incontinence pad may further comprise the step of providing said absorbing core in a form comprising a layer of water-absorbing felt and at least one water-permeable fabric arranged at one side of the water-absorbing felt, and possibly a further water-permeable fabric arranged at the other side of the water-absorbing felt.

The method for production of an incontinence pad may further comprise the step of providing said absorbing core comprising sewing one, two or more liquid-permeable membranes.

The method for production of an incontinence pad may further comprise the following steps:
Providing said absorbing core comprising sewing together at least a first part of the absorbing core and a second part of the absorbing core.

Sewing together at least a first part of the absorbing core and a second part of the absorbing core shaping the absorbing core into a form which can accommodate male genitalia. The form is preferably provided cup-formed.

Sewing together a first part of the circumference of the liquid-impermeable membrane and a second partof the circumference of the liquid-impermeable membrane defining a second sewing. The second sewing is shaping the liquid-impermeable membrane into a form which can accommodate male genitalia. The form is preferably provided cup-formed.

The method for production of an incontinence pad may further comprise the step of providing an auxiliary liquid-impermeable membrane, which is sewn to the absorbing core, in a position where said auxiliary liquid-impermeable membrane is placed between the absorbing core and the liquid-impermeable membrane in the final product, which auxiliary liquid-impermeable membrane overlaps with a part of or the whole second sewing.

The auxiliary liquid-impermeable membrane may cover part of or an entire second sewing in the liquid-impermeable membrane. By arranging the liquid-impermeable membrane and the auxiliary liquid-impermeable membrane in this way, an improved liquid seal is provided in the presence of the second sewing in the liquid-impermeable membrane.

The method for production of an incontinence pad may further comprise the step of sealing at least one part of a first sewing to make it liquid-impermeable, for instance by gluing.

An aspect of the invention may be an incontinence pad, which has been produced according to any of the previously described methods.

An aspect of the invention may be the use of a product according to any of the previous descriptions in a pair of briefs.

An aspect of the invention may be the use of a product according to any of the previous descriptions together with a liquid-proof bag, which bag can be sealed and used for storage of said product.

The incontinence pad is washable and reusable, said incontinence pad comprising an absorbing core, a liquid-impermeable membrane, and a first sewing, both the absorbing core and the liquid-impermeable membrane are produced from textile materials, said absorbing core and liquid-impermeable membrane being arranged to form together a waterproof incontinence pad for collection and storage of liquid, wherein the absorbing core and the liquid-impermeable membrane are sewn together along the edges thereof in such a way that the liquid-impermeable membrane encloses an edge area on an circumference of the absorbing core so that liquid is prevented from leaving the incontinence pad through the edge area or the backside of the absorbing core.

The use of known textile materials allows for a factory-tailoring of an incontinence pad, which is water-proof, dry and comfortable in use.

By sewing the absorbing core and the liquid-impermeable membrane this way, it can be ensured that the water-impermeable fabric of the liquid-impermeable membrane completely encloses the absorbing core, thus forming a waterproof reservoir around it.

Furthermore, the incontinence pad according to the present invention can be washed and reused a considerable amount of times, minimum 120 times, which means that significant cost savings can be obtained, compared to the use of more traditional pads, which are made from paper materials.

Even further, the risk of allergic reactions and/or skin eruptions is reduced when using known and proven textile materials.

In an embodiment, the absorbing core comprises a layer of a water-absorbing felt arranged between two layers of water-permeable fabric, one of which forms the topside of the absorbing core and of the incontinence pad.

In an embodiment, the water-absorbing felt is polyester or a similar highly absorbent textile material.

In an embodiment, the water-permeable fabric forming the topside of the absorbing core and of the incontinence pad is polyester or a similar textile material, which is rapidly penetrated by liquids and which rapidly dries out.

In an embodiment, the water-permeable fabric not forming the topside of the absorbing core and the incontinence pad is a microfilament fabric or a similar highly absorbent textile material.

In an embodiment, the water-absorbing felt is sewn together with the two layers of water-permeable fabric.

Such a configuration and choice of materials for the upper part ensures that liquid is fast and effectively absorbed by the incontinence pad leaving the topside facing the user dry and comfortable.

In an embodiment, the liquid-impermeable membrane is produced from a water-impermeable fabric.

In an embodiment, the water-impermeable fabric is cotton, polyester, elastane or mixtures thereof, which is coated on one side with a layer of a water-resistant material.

In an embodiment, the water-resistant material is rubber or a plastic material.

Such a configuration and choice of materials for the liquid-impermeable membrane ensures that the liquid is kept within the incontinence pad, ensuring that the surrounding briefs and other clothes are kept dry and comfortable.

In an embodiment, the shape and size of the incontinence pad is adapted to have the most comfortable fit for either men or women.

The adaptation of the shape and size of the incontinence pad to be used by either men or women means that both comfort and function of the incontinence pad can be optimized for both genders, respectively.

Furthermore, such gender-adapted incontinence pads can be made to fit optimally into men's or women's briefs, respectively.

In an embodiment, the incontinence pad further comprises one or more press studs arranged on the outer surface of the liquid-impermeable membrane to engage with one or more counterpart press studs located in a pair of briefs for securing the incontinence pad in the briefs.

The use of press studs means that the incontinence pad can easily and reliably be secured within the briefs for maintaining the maximum comfort and the best function of the incontinence pad during use.

In an aspect of the invention, a pair of factory-tailored briefs comprises one or more press studs arranged on its inside surface to engage with one or more counterpart press studs located on an incontinence pad, as described above, for securing the incontinence pad in the briefs, which press studs are invisible from the outside of the briefs.

### Description of the Drawing

In the following, a few exemplary embodiments of the invention are described in further detail with reference to the figures, of which:
- Figs. 1-8: schematically illustrate the most important steps of a process for pro-ducing a female incontinence pad, according to the described method and a first embodiment of the invention,
- Fig. 9: shows the produced female incontinence pad as seen from the upper side,
- Fig. 10: shows the same female incontinence pad as seen from the underside,
- Figs. 11-23: schematically illustrate the most important steps of the described method for producing a male incontinence pad, according to a second embodiment of the invention,
- Fig. 24: shows the produced male incontinence pad as seen from the outer side,
- Fig. 25: shows the same male incontinence pad as seen from the inner side,
- Fig. 26: shows the same male incontinence pad in a perspective view,
- Fig. 27: schematically shows a pair of briefs for use with an incontinence pad, according to an embodiment of the invention,
- Fig. 28: schematically illustrates how an incontinence pad, according to an embodiment of the invention, is mounted within a pair of briefs,
- Fig. 29: schematically shows a pair of briefs with a male incontinence pad, according to an embodiment of the invention,
- Fig. 30: schematically shows how a used incontinence pad, according to an embodiment of the invention, is removed from a pair of briefs,
- Fig. 31: schematically shows a pair of briefs with a female incontinence pad, according to an embodiment of the invention,
- Fig. 32: schematically shows how a used incontinence pad, according to an embodiment of the invention, can be folded together and put into a sealable bag,
- Fig. 33: schematically shows how the bag is sealed to form a water-proof bag, and
- Fig. 34: schematically shows how the sealable bag can be opened and the incontinence pad can be washed in a washing machine.

In the following text, the figures will be described one by one and the different parts and positions seen in the figures will be numbered with the same numbers in the different figures. Not all parts and positions indicated in a specific figure will necessarily be discussed together with that figure.

### List of reference numbers

1. Female incontinence pad
2. Absorbing core of female incontinence pad
3. Water-permeable fabric
4. Water-absorbing felt
5. Water-impermeable fabric
6. Liquid-impermeable membrane for female incontinence pad
7. Water-proof press stud
8. Male incontinence pad
9. Absorbing core of male incontinence pad
10. Cut line
11. Liquid-impermeable membrane for male incontinence pad
12. Briefs
13. Water-proof bag
14. Washing machine
15. Circumference of absorbing core
16. Topside
17. Backside
18. Height of the core
19. Distance between first sewing and the circumference of the absorbing core
20. First sewing
21. Circumference of the liquid-impermeable membrane
22. First part of the circumference of the absorbing core
23. Second part of the circumference of the absorbing core
24. Hole
25. First part of the absorbing core
26. Second part of the absorbing core
27. Part A of the circumference of the liquid-impermeable membrane
28. Part B of the circumference of the liquid-impermeable membrane
29. Folds
30. Auxiliary liquid-impermeable membrane
31. Second sewing
All non-marked dashed lines indicate a sewing.

### Detailed Description of the Invention

In order to produce environment- and skin-friendly products 1, 8, 12 to incontinence patients from washable textile pillows, a certain challenge must be overcome, namely to produce a liquid-impermeable membrane 6, 11 from a water-impermeable fabric 5 and sew it together with a water-permeable fabric 3 without leaving any stitch holes in the liquid-impermeable membrane 6, 11. Just a single stitch hole in the liquid-impermeable membrane 6, 11 made from the water-impermeable fabric 5 can allow liquid to penetrate the liquid-impermeable membrane 6, 11 with severe consequences for the user.

Thus, it has been necessary to develop a new sewing technique in order to be able to join a liquid-impermeable membrane 6, 11 from a water-impermeable fabric 5, with an absorbing core, which renders the final product absolutely waterproof and stays water-proof. The liquid-impermeable membrane 6, 11 is sewn together with the absorbing core 2, 9 comprising a water-permeable fabric 3, through which liquid must be able to seep through and into the absorbing core.

The liquid-impermeability of the incontinence pad is achieved in this new sewing technique, by providing a liquid-impermeable membrane 6, 11 with larger dimensions than the absorbing core 2, 9. The circumference of the liquid-impermeable membrane 6, 11 and the absorbing core 2, 9 are then aligned and sewed together in a distance 19 between the first sewing and the circumference of the absorbing core. Thus, when the workpiece is turned inside out in the process, the liquid-impermeable membrane covers the parts of the absorbing core necessary for providing a liquid-impermeable membrane in the relevant parts of the incontinence pads, namely along the backside 17, stretching over the height 18 of the core, and including an edge area between the first sewing 20 and the circumference 15 of the absorbing core 2, 9.

In the following, the production of some examples of incontinence pads 1, 8 are described, according to the invention, using the new sewing method.

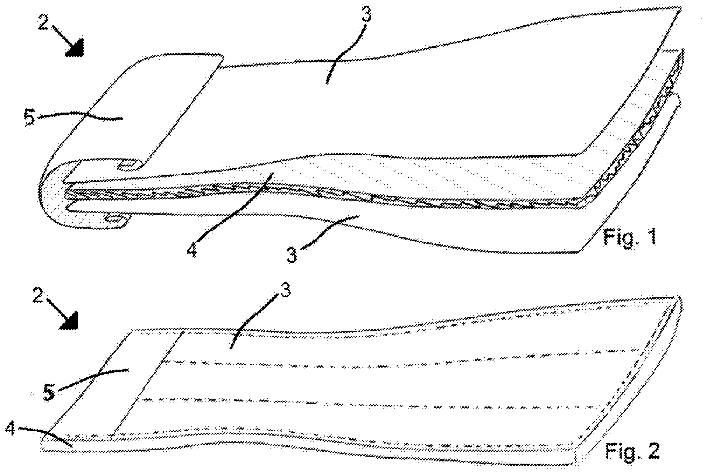

Figs. 1-8 schematically illustrate the most important steps of a process for producing a female incontinence pad 1, according to a first embodiment of the invention.

Figs. 1 and 2 illustrate how two pieces of water-permeable fabric 3 are placed on opposite sides, respectively, of a piece of water-absorbing felt 4 and the three layers are sewn together to form an absorbing core 2 of the female incontinence pad.

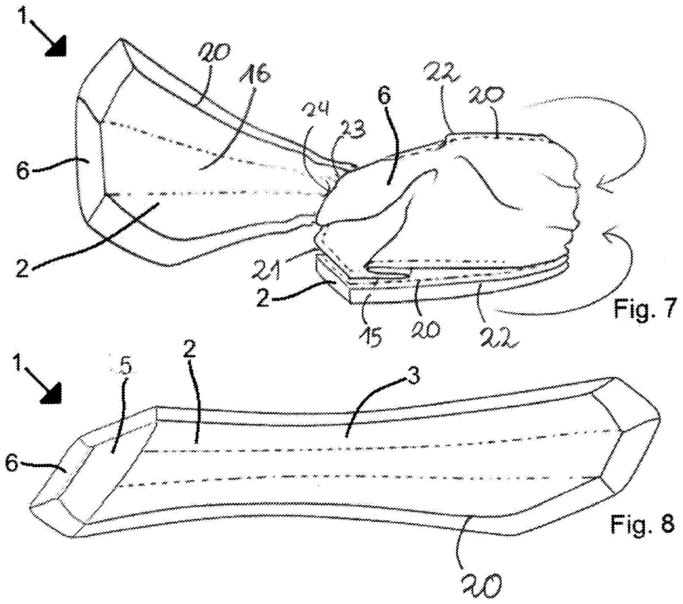

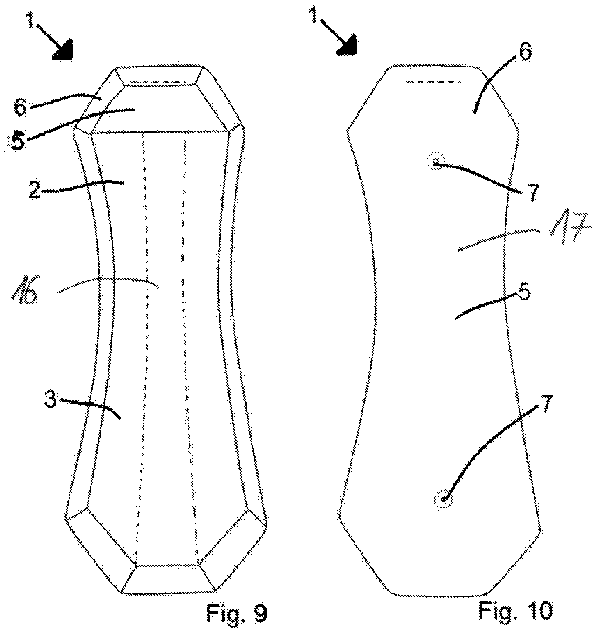

A piece of water-impermeable fabric 5 is folded around one end of the water-permeable fabrics 3 and the water-absorbing felt 4 to form the frontmost end of the water absorbing core 2, which works as a form of lid for the liquid reservoir 6 as seen in Figs. 8 and 9. The part of the absorbing core 2 which is not covered by the water- impermeable fabric 5 forms the topside 16 of the absorbing core 2 and of the female incontinence pad 1, through which liquid can seep into the absorbing core 2 and be retained inside the liquid-impermeable membrane 6 when the incontinence pad 1 is used.

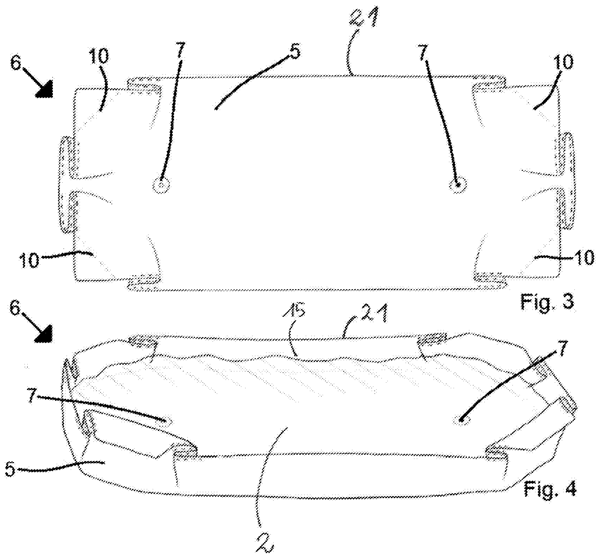

Figure 3 shows how the liquid-impermeable membrane 6 formed by a water-impermeable fabric 5 is brought into shape for alignment with the absorbing core 2 by folding and sewing it appropriately. The four corners are cut off along the indicated cut lines 10 and two water-proof press studs 7 are mounted within the liquid-impermeable membrane 6.

Figure 4 schematically shows the difference in size of the absorbing core 2 and the liquid-impermeable membrane 6. The liquid-impermeable membrane 6 can encompass the entire circumference 15 of the absorbing core and form a liquid-proof barrier around the absorbing core 2 without any stitch holes in the liquid-holding part.

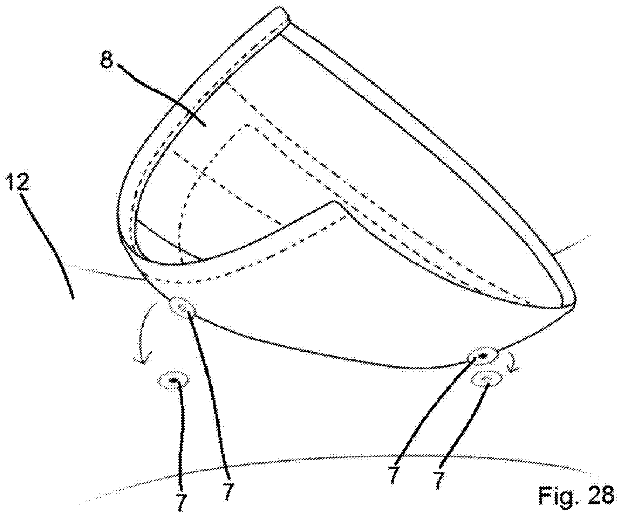

Preferably, the press studs 7 are oriented in two opposite directions, i.e. there is one male part and one female part. In this way, it can be ensured that the incontinence pad 1 will always be oriented correctly when mounted in a pair of briefs 12 according to the invention as shown in Fig. 28.

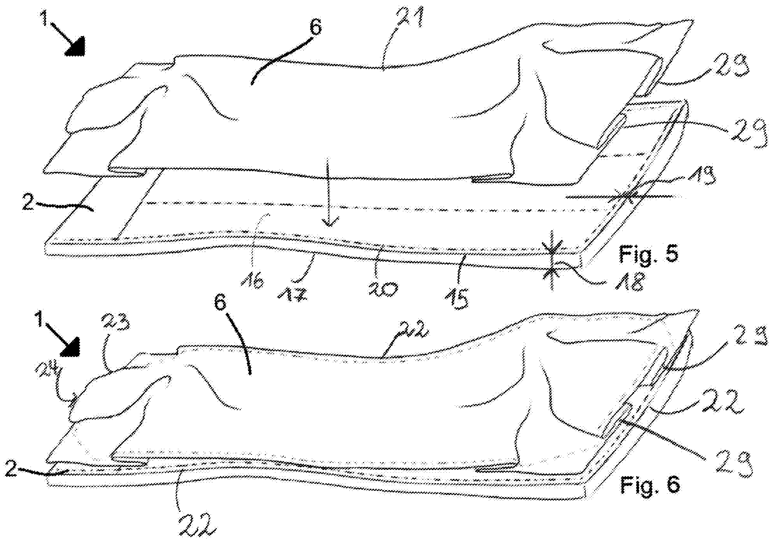

Figs. 5 and 6 illustrate how the liquid-impermeable membrane 6 is placed against the absorbing core 2. The topside 16 of the absorbing core 2 faces the liquid-impermeable membrane. The two parts 2, 6 are sewn together along the first sewing 20 in a distance 19 between first sewing 20 and the circumference 15 of the absorbing core 2, along a first part 22 of the circumference 15 of the absorbing core 2, "cutting" the corners. The excessive water-impermeable fabric 5 of the liquid-impermeable membrane 6 in the corners outside of a distance 19 from the first sewing are cut off, as well as the corresponding excessive part of the absorbing core 2. The second part 23 of the circumference 15 of the absorbing core 2 is left non-sewn.

Figure 7 illustrates how the female incontinence pad 1 is turned inside out through the non-sewn hole 24. This leads to the liquid-impermeable membrane 6 encompassing the backside 17 of the absorbing core, the height 18 of the core and an edge area in a distance 19 between the first sewing 20 and the circumference 15 of the absorbing core 2 on the topside 16 of the absorbing core 2.

Figure 8 shows the workpiece after turning the inside out. The non-sewn part of the liquid-impermeable membrane 6 in the second part 23 of the circumference 15 of the absorbing core 2 is sewn to said absorbing core 2. This last sewing is placed at a position where there are many layers of water-impermeable fabric 5, which ensures that no liquid will be able to penetrate the liquid-impermeable membrane 6 through the stitching holes of this sewing.

Following the process described above results in the female incontinence pad 1, which is shown from the upper side and the underside, respectively, in Figs. 9 and 10. Fig. 9 illustrates how the edge of the liquid-impermeable membrane 6 encloses the edge of the absorbing core 2 completely. This ensures that any liquid stored within the water-absorbing felt material 4 of the absorbing core 2 and in the liquid-impermeable membrane 6 is prevented from leaving the female incontinence pad 1 through the edge thereof.

As mentioned above, the topside 16 of the absorbing core 2 and the female incontinence pad 1 is covered with a layer of water-impermeable fabric 5 at a second part 23 of the circumference of the absorbing core 2, which works as a "lid" for this part of the absorbing core 2 in order to increase the liquid-holding capacity of the female incontinence pad.

The described special sewing method with folding of the material especially in the corners of the female incontinence pad 1 ensures softness of the edges and comfort for the user while still being absolutely waterproof.

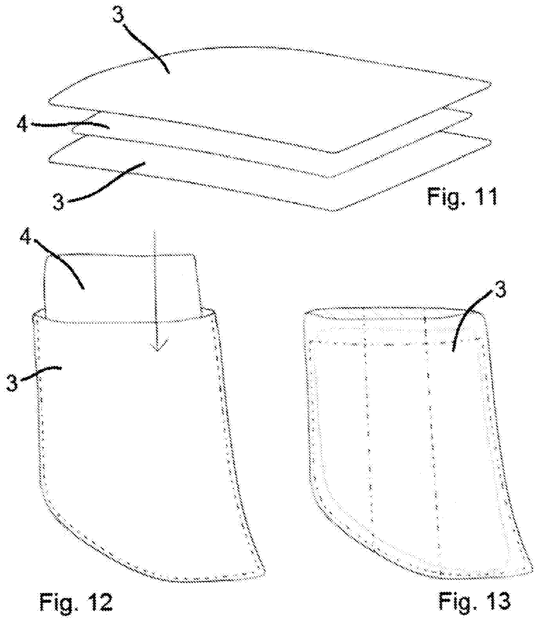

Figs. 11-23 schematically illustrate the most important steps of a process for producing a male incontinence pad 8, according to a second embodiment of the invention.

Figs. 11-13 illustrate a part of producing the absorbing core: how two pieces of water-permeable fabric 3 are sewn together to form a pocket, into which a piece of water-absorbing felt 4 is placed, before the pocket and the water-absorbing felt 4 therein are sewn together as illustrated in Fig. 13.

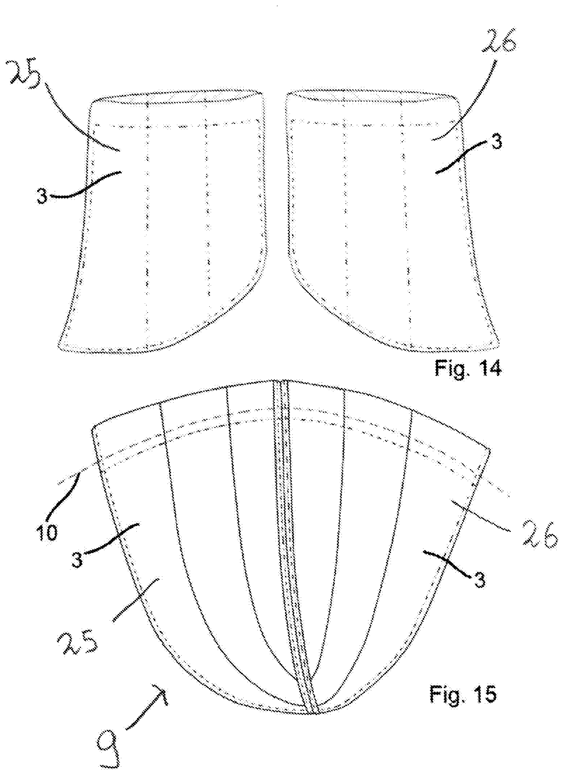

Figs. 14 and 15 illustrate a part of producing the absorbing core: how two such sewn-together pockets, the first part 25 of the absorbing core and the second part 26 of the absorbing core, are mirrored and placed against each other before they are sewn together and the excessive water-permeable fabric 3 is cut off along the indicated cut line 10.

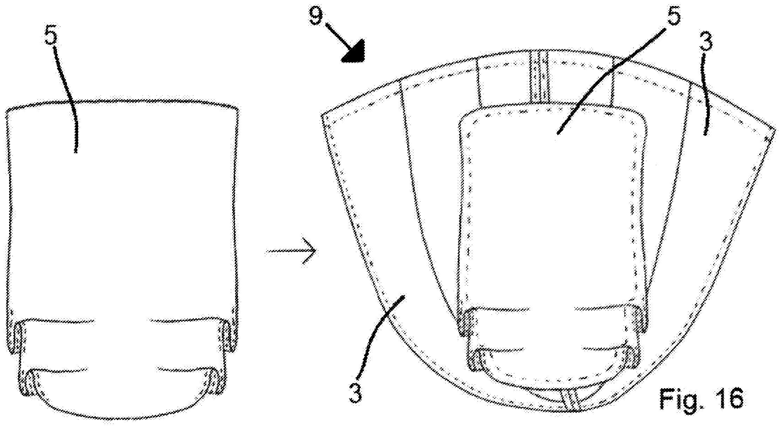

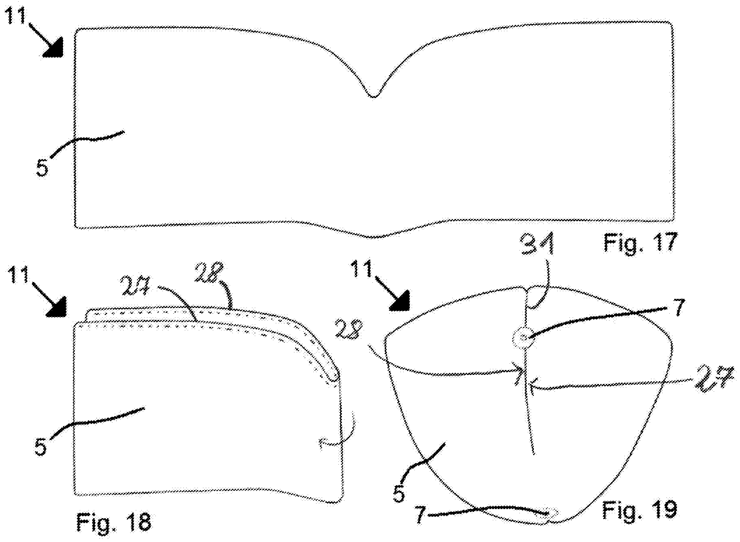

Fig. 16 illustrates how a piece of water-impermeable fabric 5 is folded and stitched at the edges to form a rounded piece without stitch holes where the liquid is to be stored. This piece is sewn onto the absorbing core 9 shown in Fig. 15 to give an additional layer of liquid-impermeability in the male incontinence pad 8 as illustrated in Fig. 16, especially when the liquid-impermeable membrane 11 comprises a second sewing as shown in Figs. 17-19.

Figs. 17-19 illustrate how the liquid-impermeable membrane 11 of the male incontinence pad 8 is formed by cutting out a piece of water-impermeable fabric 5 (Fig. 17), folding it, so that the part A 27 of the circumference 21 of the liquid-impermeable membrane 11 and the part B 28 of the circumference 21 of the liquid-impermeable membrane 11 are aligned, and sewing it together (Fig. 18), turning it inside out and mounting two water-proof press studs 7 therein (Fig. 19). For the same reason as mentioned above for the female incontinence pad 1, the two press studs 7 are preferably oppositely directed, i.e. there is one male part and one female part.

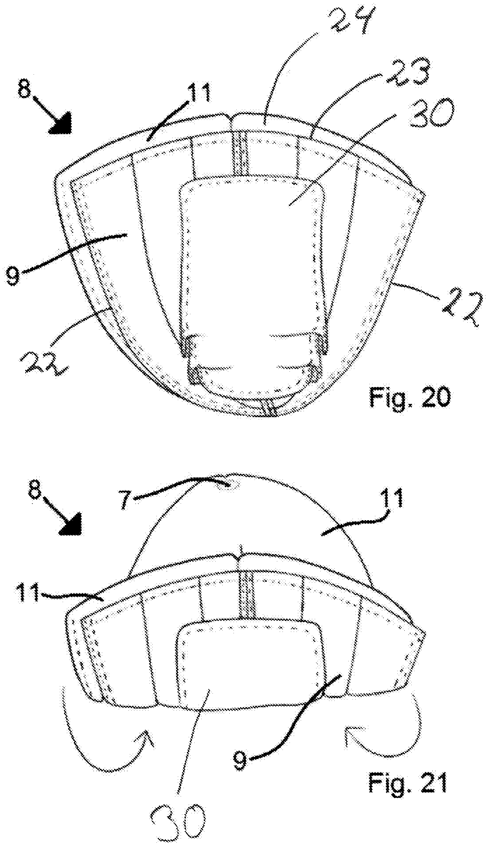

Figs. 20 and 21 illustrate how the male incontinence pad 8 is formed by sewing together the absorbing core 9 and the liquid-impermeable membrane 11 thereof, leaving a non-sewn hole 24 (the upper edge in Fig. 20), through which the male incontinence pad 8 is turned inside out.

An auxiliary liquid-impermeable membrane 30 can be sewn to the absorbing core 9in a position where said auxiliary liquid-impermeable membrane 30 is placed between the absorbing core 9 and the liquid-impermeable membrane 11 in the final product. The auxiliary liquid-impermeable membrane 30 overlaps with a part of or the whole second sewing 31.

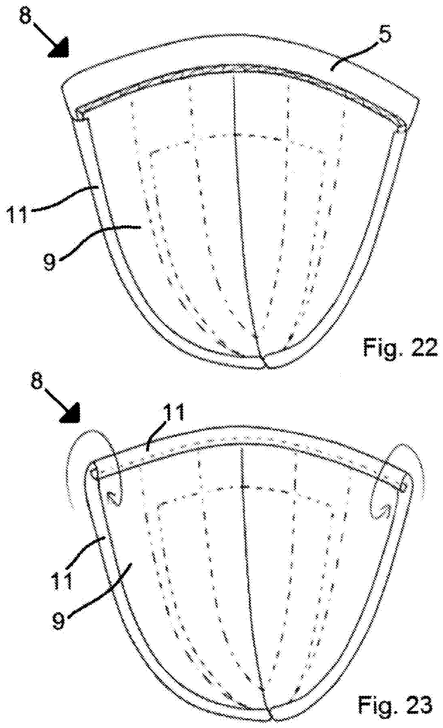

Figs. 22 and 23 illustrate how the liquid-impermeable membrane in the second part 23 of the circumference 15 of the absorbing core 9 is folded around the height 18 of and over an edge area of the absorbing core 9 and sewn together.

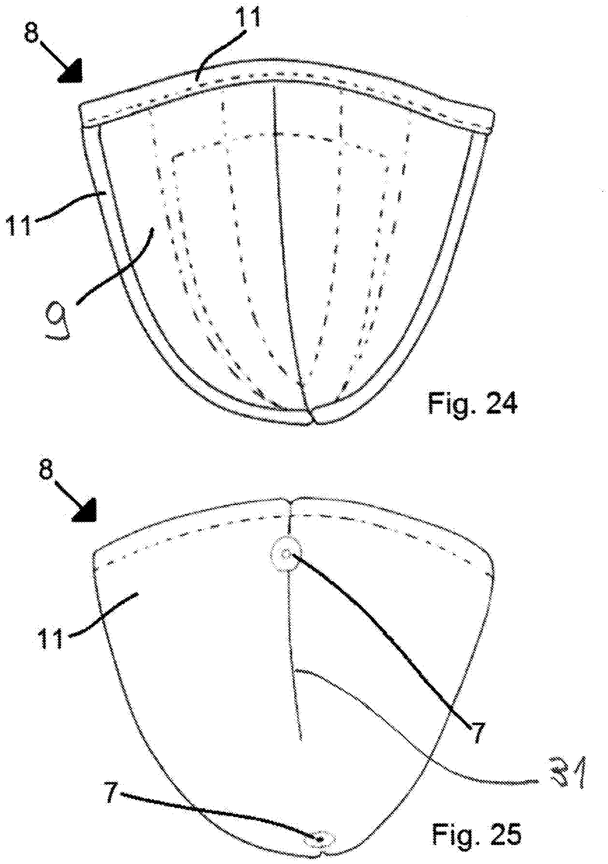

Following the above-described process results in the male incontinence pad 8, which is shown from the inner side, from the outer side and in a perspective view, respectively, in Figs. 24-26. Fig. 24 illustrates how the edge of the liquid-impermeable membrane 11 encloses the entire circumference 15 of the absorbing core 9 and an edge area up until the first sewing 20 of the absorbing core 9 completely. This ensures that any liquid stored within the water-absorbing felt material 4 of the absorbing core 9 is prevented from leaving the male incontinence pad 8 through the circumference 15 of the absorbing core 9.

The male incontinence pad 8 works as a liquid-impermeable vessel, which is able to keep the liquid inside itself. The male incontinence pad 8 is arranged to be placed more forward and upward in a pair of briefs 12 than the female incontinence pad 1.

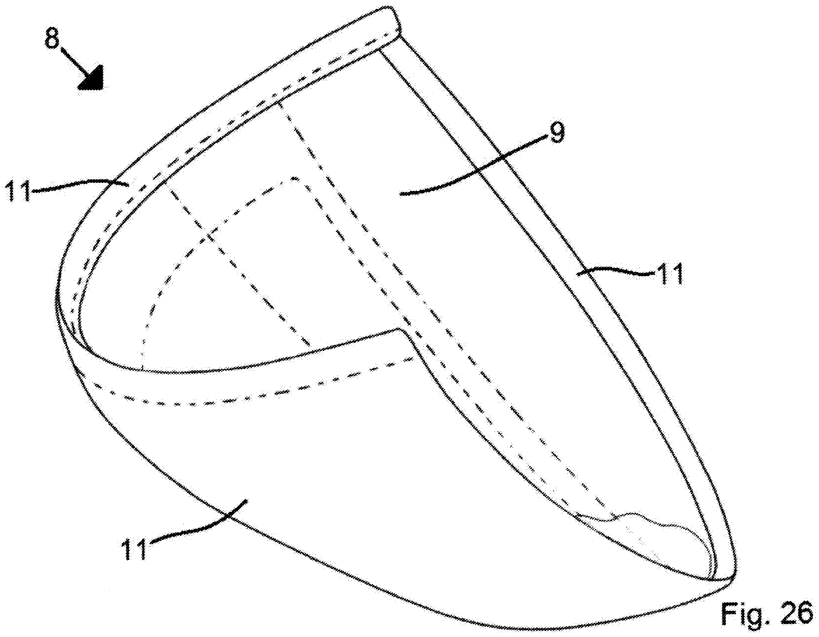

Apart from the triangular shape, there is another main feature distinguishing the male incontinence pad 8 from the female incontinence pad 1: as indicated especially in Figs. 26 and 28, the male incontinence pad 8 produced as described above is pre-shaped with a certain bend along its longitudinal axis providing a more anatomic and more comfortable shape of the male incontinence pad 8 for male users.

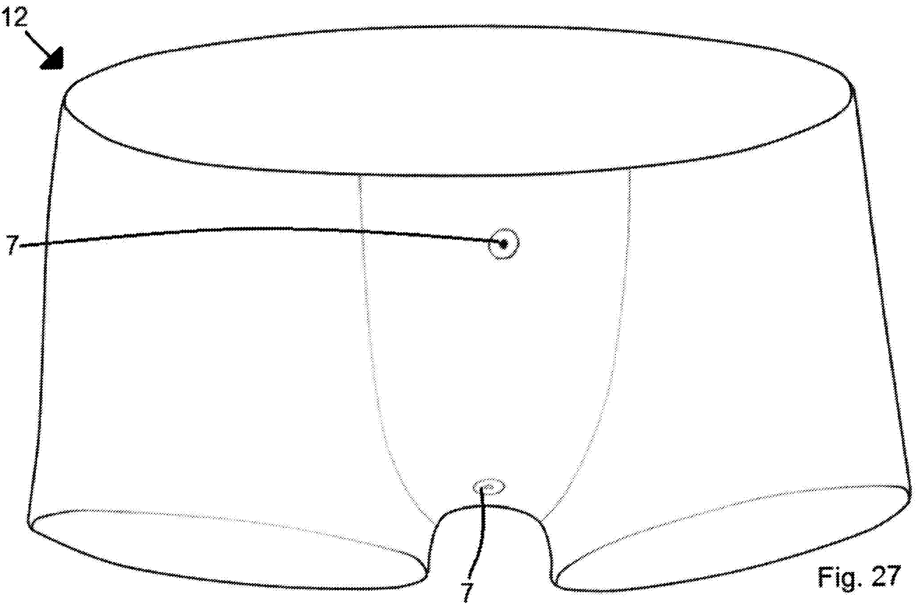

Fig. 27 schematically shows a pair of briefs 12 with a couple of press studs 7 mounted therein. For the sake of ensuring the correct orientation of an incontinence pad 1, 8 mounted within the briefs 12 and indicated in Fig. 28, the two press studs 7 are preferably oppositely directed, i.e. there is one male part and one female part.

Fig. 28 schematically illustrates how a male incontinence pad 8 is mounted within a pair of briefs 12 by assembling the press studs 7 of the male incontinence pad 8 and with the corresponding ones of the briefs 12. As indicated in the figure, making sure that the two press studs 7 of the male incontinence pad 8, and the briefs 12 are oppositely directed as described above, a correct orientation of the male incontinence pad 8 within the briefs 12 is ensured. A female incontinence pad 1 is mounted in a pair of briefs 12 in the same way.

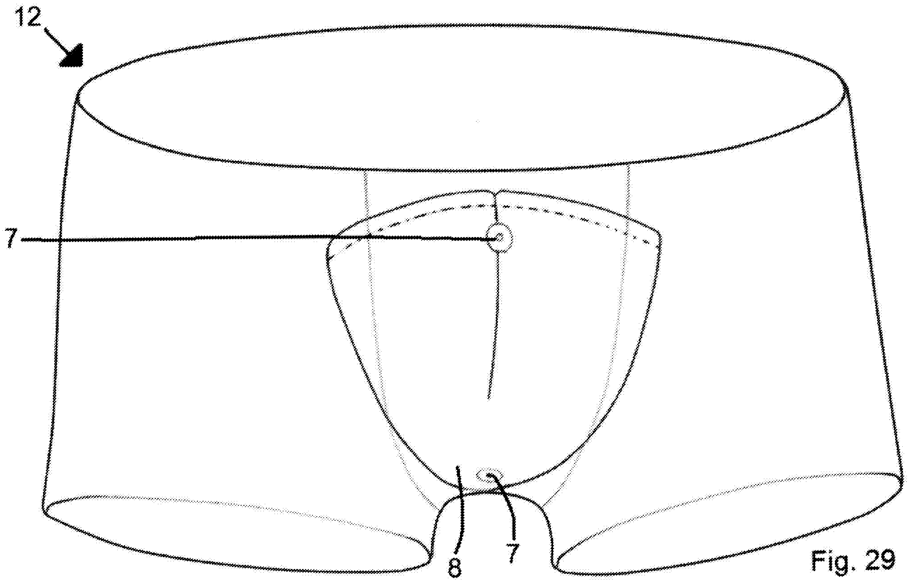

Fig. 29 schematically shows a pair of briefs 12 with a male incontinence pad 8 mounted therein.

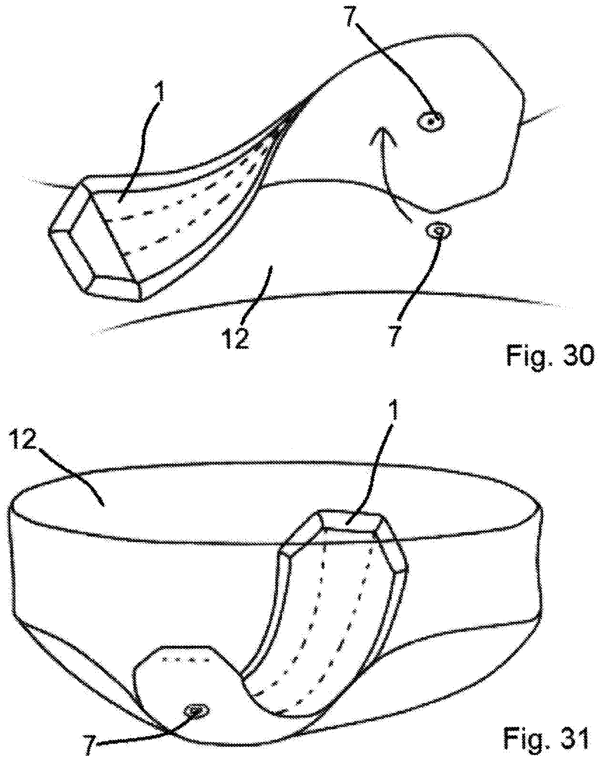

Fig. 30 schematically shows how a used female incontinence pad 1 is removed from a pair of briefs 12 by disassembling the press studs 7 holding the two parts 1, 12. A male incontinence pad 8 is removed from a pair of briefs 12 in the same way.

Fig. 31 schematically shows a pair of briefs 12 with a female incontinence pad 1 mounted therein.

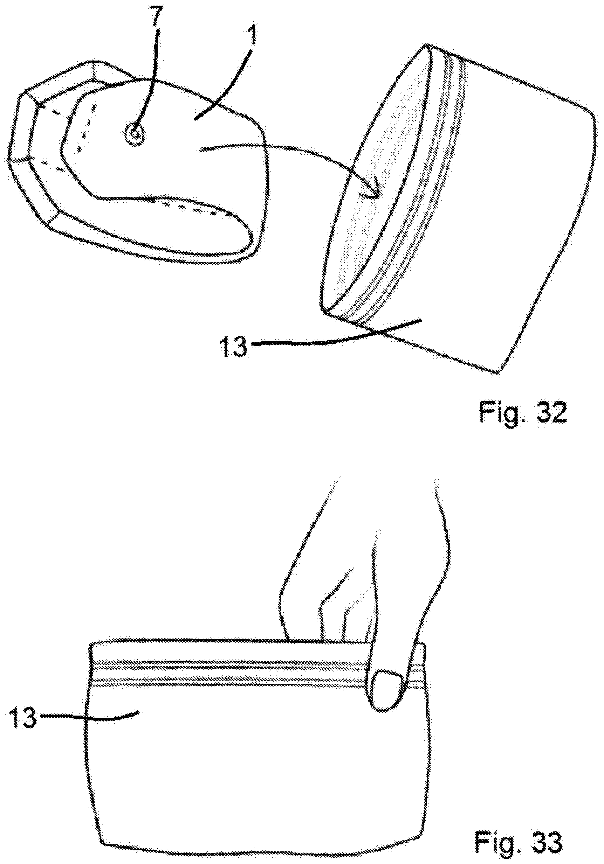

Figs. 32 and 33 schematically show how a used female incontinence pad 1 can be folded together and put into a water-proof bag 13, which can then be sealed and stored until the female incontinence pad 1 is to be washed. Obviously, a male incontinence pad 8 can be folded and stored in a water-proof bag 13 in the same way. Preferably, disposable water-proof bags 13, each holding a single used incontinence pad 1, 8, are used but, if so desired, resealable and reusable bags can, of course, also be used.

Preferably, the used incontinence pad 1, 8 is folded with the water-impermeable fabric 5 forming the liquid-impermeable membrane 6, 11 facing outwards. In this way, any liquid is kept inside the incontinence pad 1, 8. If stored in a waterproof sealed bag 13, the used incontinence pad 1, 8 can be stored at least up till two weeks without drying out, which means that it can still be washed clean and reused.

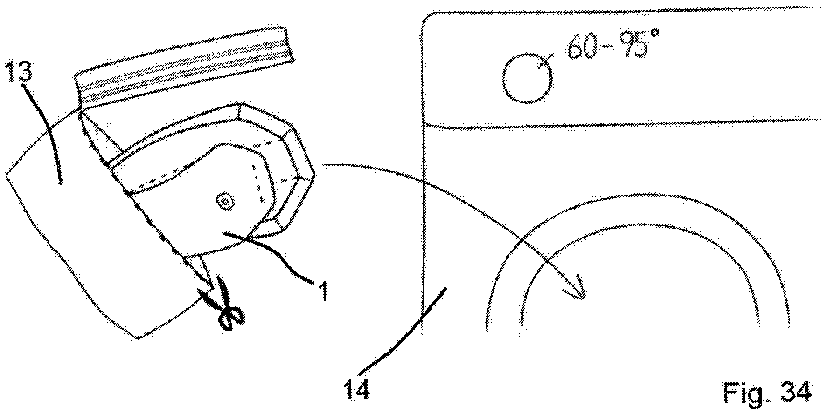

Fig. 34 schematically shows how a water-proof bag 13 can be opened, for instance, by cutting it open and the incontinence pad 1, 8 therein can be washed in a washing machine 14 at 60-95 °C. Due to the fact that used incontinence pads 1, 8 can be stored for a long time before washing, many incontinence pads 1, 8 can be washed and together. The incontinence pads 1, 8 can be dried in a tumbler or hanging in a laundry bag. Each incontinence pad 1, 8 may be reused and washed up till 120 times.

## Claims

1. A method for production of an incontinence pad (1, 8) comprising an absorbing core (2, 9), a liquid-impermeable membrane (6, 11), and a first sewing (20) connecting the absorbing core (2, 9) and the liquid-impermeable membrane (6, 11), which method comprises at least the following steps:
- providing an absorbing core (2, 9) with a circumference (15) of the absorbing core (2, 9), a topside (16), a backside (17), and a height of the core (18), where the topside (16) is oriented towards the user and the backside is oriented away from the user when said incontinence pad (1, 8) is in use,
- providing a liquid-impermeable membrane (6, 11),
- placing said liquid-impermeable membrane (6, 11) on top of said absorbing core (2, 9), with the topside (16) of the core facing said liquid-impermeable membrane (6, 11), aligning said circumference (15) of the absorbing core (2, 9) and said circumference (21) of the liquid-impermeable membrane (6, 11),
- sewing together said absorbing core (2, 9) and said liquid-impermeable membrane (6, 11) along a first part (22) of the circumference (15) of the absorbing core (2, 9), thereby leaving a second part (23) of the circumference (15) of the absorbing core (19) non-sewn, defining a hole (24) between the connected absorbing core (2, 9) and the liquid-impermeable membrane (6, 11), and
- turning the inside out of the product through the hole (24),
**characterized in that** said liquid-impermeable membrane (6, 11) has at least the dimensions of the backside (17) of said absorbing core (2, 9), plus the height (18) of the core, plus twice the distance (19) of said first sewing (20) to said circumference (15) of the absorbing core (2, 9), and said first part (22) of the circumference (15) of the absorbing core (2, 9) constitutes an essential part of the circumference (15) of the absorbing core (2, 9), such that after turning the inside out of the product through the hole (24), the liquid-impermeable membrane (6, 11) will encompass the backside (17) of the absorbing core (2,9), the height (18) of said absorbing core (2, 9) and inwards over an edge area corresponding to the sewing distance (19) from the circumference (15) of said absorbing core (2, 9), and wherein said method further comprises at least the following steps:- folding the non-sewn part of the liquid-impermeable membrane (6, 11) up along the height (18) of and over the absorbing core's (2, 9) edge region in said second part (23) of the circumference (15) of the absorbing core (2, 9), and
- sewing of the non-sewn part of the liquid-impermeable membrane (6, 11) to the absorbing core (2, 9) in said second part (23) of the circumference (15) of the absorbing core (2, 9).

2. A method for production of an incontinence pad (1, 8) according to claim 1, **characterized in that** said method further comprises the following steps:
- providing an additional layer of the liquid-impermeable membrane, which is folded over in said second part (23) of the circumference (15) of the absorbing core (2, 9) and sewing the folded over additional layer of the liquid-impermeable membrane across said second part (23) of the circumference (15) of the absorbing core (2, 9), so that the folded over liquid-impermeable membrane is sewn onto the topside and backside of the absorbing core.

3. A method for production of an incontinence pad (1, 8) according to any of the previous claims, **characterized in that** said method further comprises the following steps:
- folding the liquid-impermeable membrane (6, 11) into folds (29) in areas located close to corners in the absorbing core (2, 9) when the circumference (15) of the absorbing core (2, 9) and the circumference (21) of the liquid-impermeable membrane (6, 11) are brought into alignment,
- sewing of said folds (29).

4. A method for production of an incontinence pad (1, 8) according to any of the previous claims, **characterized in that** said method further comprises the following step:
- providing said liquid-impermeable membrane (6, 11) with at least two water-proof press-studs (7).

5. A method for production of an incontinence pad (1, 8) according to any of the previous claims, **characterized in that** said method further comprises the following steps:
- providing said absorbing core (2, 9) in a form comprising a layer of water-absorbing felt (4) and at least one water-permeable fabric (3) arranged at one side of the water-absorbing felt (4), and possibly a further water-permeable fabric (3) arranged at the other side of the water-absorbing felt (4).

6. A method for production of an incontinence pad (1, 8) according to any of the previous claims, **characterized in that** said method further comprises the following steps:
- providing said absorbing core (2, 9) comprising sewing one, two or more water-permeable fabrics (3).

7. A method for production of an incontinence pad (1, 8) according to any of the previous claims, **characterized in that** said method further comprises the following steps:
- providing said absorbing core (2, 9) comprising sewing together at least a first part (25) of the absorbing core (2, 9) and a second part (26) of the absorbing core (2, 9),
- sewing together a first part (27) of the circumference (21) of the liquid-impermeable membrane (6, 11) and a second part (28) of the circumference (21) of the liquid-impermeable membrane (6, 11) defining a second sewing (31), which second sewing is shaping the liquid-impermeable membrane into a form which can accommodate male genitalia.

8. A method for production of an incontinence pad (1, 8) according to claim 7, **characterized in that** said method further comprises the following steps:
- providing an auxiliary liquid-impermeable membrane (30), which is sewn to the absorbing core (2, 9), in a position where said auxiliary liquid-impermeable membrane (30) is placed between the absorbing core (2, 9) and the liquid-impermeable membrane (6, 11) in the final product, which auxiliary liquid-impermeable membrane (30) overlaps with a part of or the whole second sewing (31).

9. An incontinence pad comprising an absorbing core (2, 9) with a circumference (15) of the absorbing core (2, 9), a topside (16), a backside (17), and a height of the core (18), where the topside (16) is oriented towards the user and the backside is oriented away from the user when said incontinence pad (1, 8) is in use, a liquid-impermeable membrane (6, 11), and a first sewing (20) connecting the absorbing core (2, 9) and the liquid-impermeable membrane (6, 11) along a first part (22) of the circumference (15) of the absorbing core (2, 9), and a second sewing connecting the liquid-impermeable membrane (6, 11) to the absorbing core (2, 9) in a second part (23) of the circumference (15) of the absorbing core (2, 9),
wherein said liquid-impermeable membrane (6, 11) is placed on top of said absorbing core (2, 9), with the topside (16) of the core facing said liquid-impermeable membrane (6, 11), and said circumference (15) of the absorbing core (2, 9) aligned with said circumference (21) of the liquid-impermeable membrane (6, 11),
**characterized in that** said liquid-impermeable membrane (6, 11) has at least the dimensions of the backside (17) of said absorbing core (2, 9), plus the height (18) of the core, plus twice the distance (19) of said first sewing (20) to said circumference (15) of the absorbing core (2, 9), and wherein said liquid-impermeable membrane (6, 11) encompasses the backside (17) of the absorbing core (2,9), the height (18) of said absorbing core (2, 9) and an edge area corresponding to the sewing distance (19) from the circumference (15) of said absorbing core (2, 9).

10. An incontinence pad according to claim 9 **characterized in that** it has been produced with a method according to any one or more of claims 1-8.

11. The use of an incontinence pad according to any one of claims 9 or 10 in a pair of briefs.

12. The use of an incontinence pad according to any one of claims 9 or 10 together with a liquid-proof bag, which bag can be sealed and used for storage of said product.

## Patentansprüche

1. Verfahren zur Herstellung einer Inkontinenzeinlage (1, 8), umfassend einen absorbierenden Kern (2, 9), eine flüssigkeitsundurchlässige Membran (6, 11) und eine erste Naht (20), die den absorbierenden Kern (2, 9) und die flüssigkeitsundurchlässige Membran (6, 11) verbindet, wobei das Verfahren mindestens die folgenden Schritte umfasst:
- Bereitstellen eines absorbierenden Kerns (2, 9) mit einem Umfang (15) des absorbierenden Kerns (2, 9), einer Oberseite (16), einer Rückseite (17) und einer Höhe des Kerns (18), wobei die Oberseite (16) zum Benutzer hin ausgerichtet ist und die Rückseite vom Benutzer weg ausgerichtet ist, wenn die Inkontinenzeinlage (1, 8) in Verwendung ist,
- Bereitstellen einer flüssigkeitsundurchlässigen Membran (6, 11),
- Platzieren der flüssigkeitsundurchlässigen Membran (6, 11) auf dem absorbierenden Kern (2, 9), wobei die Oberseite (16) des Kerns der flüssigkeitsundurchlässigen Membran (6, 11) zugewandt ist, Ausrichten des Umfangs (15) des absorbierenden Kerns (2, 9) und des Umfangs (21) der flüssigkeitsundurchlässigen Membran (6, 11),
- Zusammennähen des absorbierenden Kerns (2, 9) und der flüssigkeitsundurchlässigen Membran (6, 11) entlang eines ersten Teils (22) des Umfangs (15) des absorbierenden Kerns (2, 9), wodurch ein zweiter Teil (23) des Umfangs (15) des absorbierenden Kerns (19) nicht genäht wird, wodurch ein Loch (24) zwischen dem verbundenen absorbierenden Kern (2, 9) und der flüssigkeitsundurchlässigen Membran (6, 11) definiert wird, und
- Drehen der Innenseite des Produkts durch das Loch (24),
**dadurch gekennzeichnet, dass** die flüssigkeitsundurchlässige Membran (6, 11) mindestens die Abmessungen der Rückseite (17) des absorbierenden Kerns (2, 9) plus die Höhe (18) des Kerns plus den doppelten Abstand (19) der ersten Naht (20) zu dem Umfang (15) des absorbierenden Kerns (2, 9) aufweist und der erste Teil (22) des Umfangs (15) des absorbierenden Kerns (2, 9) einen wesentlichen Teil des Umfangs (15) des absorbierenden Kerns (2, 9) bildet, sodass die flüssigkeitsundurchlässige Membran (6, 11) nach dem Umdrehen der Innenseite des Produkts durch das Loch (24) die Rückseite (17) des absorbierenden Kerns (2, 9), die Höhe (18) des absorbierenden Kerns (2, 9) und nach innen über einen Randbereich, der dem Nähabstand (19) vom Umfang (15) des absorbierenden Kerns (2, 9) entspricht, umschließt, und wobei das Verfahren ferner mindestens die folgenden Schritte umfasst:
- Falten des nicht genähten Teils der flüssigkeitsundurchlässigen Membran (6, 11) nach oben entlang der Höhe (18) und über den Randbereich des absorbierenden Kerns (2, 9) in dem zweiten Teil (23) des Umfangs (15) des absorbierenden Kerns (2, 9), und
- Annähen des nicht genähten Teils der flüssigkeitsundurchlässigen Membran (6, 11) an den absorbierenden Kern (2, 9) in dem zweiten Teil (23) des Umfangs (15) des absorbierenden Kerns (2, 9).

2. Verfahren zur Herstellung einer Inkontinenzeinlage (1, 8) nach Anspruch 1, **dadurch gekennzeichnet, dass** das Verfahren ferner die folgenden Schritte umfasst:
- Bereitstellen einer zusätzlichen Lage der flüssigkeitsundurchlässigen Membran, die in dem zweiten Teil (23) des Umfangs (15) des absorbierenden Kerns (2, 9) umgefaltet wird, und Nähen der umgefalteten zusätzlichen Lage der flüssigkeitsundurchlässigen Membran über den zweiten Teil (23) des Umfangs (15) des absorbierenden Kerns (2, 9), so dass die umgefaltete flüssigkeitsundurchlässige Membran auf die Oberseite und die Rückseite des absorbierenden Kerns genäht wird.

3. Verfahren zur Herstellung einer Inkontinenzeinlage (1, 8) nach einem der vorhergehenden Ansprüche, **dadurch gekennzeichnet, dass** das Verfahren ferner die folgenden Schritte umfasst:
- Falten der flüssigkeitsundurchlässigen Membran (6, 11) in Falten (29) in Bereichen, die sich in der Nähe von Ecken in dem absorbierenden Kern (2, 9) befinden, wenn der Umfang (15) des absorbierenden Kerns (2, 9) und der Umfang (21) der flüssigkeitsundurchlässigen Membran (6, 11) in eine Linie gebracht werden,
- Nähen der Falten (29)

4. Verfahren zur Herstellung einer Inkontinenzeinlage (1, 8) nach einem der vorhergehenden Ansprüche, **dadurch gekennzeichnet, dass** das Verfahren ferner den folgenden Schritt umfasst:
- Bereitstellen der flüssigkeitsundurchlässigen Membran (6, 11) mit mindestens zwei wasserdichten Druckknöpfen (7).

5. Verfahren zur Herstellung einer Inkontinenzeinlage (1, 8) nach einem der vorhergehenden Ansprüche, **dadurch gekennzeichnet, dass** das Verfahren ferner die folgenden Schritte umfasst:
- Bereitstellen des absorbierenden Kerns (2, 9) in einer Form, die eine Schicht aus wasserabsorbierendem Filz (4) und mindestens ein wasserdurchlässiges Gewebe (3), das auf einer Seite des wasserabsorbierenden Filzes (4) angeordnet ist, und möglicherweise ein weiteres wasserdurchlässiges Gewebe (3), das auf der anderen Seite des wasserabsorbierenden Filzes (4) angeordnet ist, umfasst.

6. Verfahren zur Herstellung einer Inkontinenzeinlage (1, 8) nach einem der vorhergehenden Ansprüche, **dadurch gekennzeichnet, dass** das Verfahren ferner die folgenden Schritte umfasst:
- Bereitstellen des absorbierenden Kerns (2, 9), umfassend das Nähen von einem, zwei oder mehreren wasserdurchlässigen Geweben (3).

7. Verfahren zur Herstellung einer Inkontinenzeinlage (1, 8) nach einem der vorhergehenden Ansprüche, **dadurch gekennzeichnet, dass** das Verfahren ferner die folgenden Schritte umfasst:
- Bereitstellen des absorbierenden Kerns (2, 9), umfassend Zusammennähen von mindestens einem ersten Teil (25) des absorbierenden Kerns (2, 9) und einem zweiten Teil (26) des absorbierenden Kerns (2, 9),
- Zusammennähen eines ersten Teils (27) des Umfangs (21) der flüssigkeitsundurchlässigen Membran (6, 11) und eines zweiten Teils (28) des Umfangs (21) der flüssigkeitsundurchlässigen Membran (6, 11), die eine zweite Naht (31) definieren, wobei die zweite Naht die flüssigkeitsundurchlässige Membran in eine Form formt, die männliche Genitalien aufnehmen kann.

8. Verfahren zur Herstellung einer Inkontinenzeinlage (1, 8) nach Anspruch 7, **dadurch gekennzeichnet, dass** das Verfahren ferner die folgenden Schritte umfasst:
- Bereitstellen einer flüssigkeitsundurchlässigen Hilfsmembran (30), die mit dem absorbierenden Kern (2, 9) vernäht ist, in einer Position, in der die flüssigkeitsundurchlässige Hilfsmembran (30) zwischen dem absorbierenden Kern (2, 9) und der flüssigkeitsundurchlässigen Membran (6, 11) im Endprodukt angeordnet ist, wobei die flüssigkeitsundurchlässige Hilfsmembran (30) mit einem Teil oder der gesamten zweiten Naht (31) überlappt.

9. Inkontinenzeinlage, umfassend einen absorbierenden Kern (2, 9) mit einem Umfang (15) des absorbierenden Kerns (2, 9), einer Oberseite (16), einer Rückseite (17) und einer Höhe des Kerns (18), wobei die Oberseite (16) zum Benutzer hin und die Rückseite vom Benutzer weg orientiert ist, wenn die Inkontinenzeinlage (1, 8) in Verwendung ist, eine flüssigkeitsundurchlässige Membran (6, 11), und eine erste Naht (20), die den absorbierenden Kern (2, 9) und die flüssigkeitsundurchlässige Membran (6, 11) entlang eines ersten Teils (22) des Umfangs (15) des absorbierenden Kerns (2, 9) verbindet, und eine zweite Naht, die die flüssigkeitsundurchlässige Membran (6, 11) mit dem absorbierenden Kern (2, 9) in einem zweiten Teil (23) des Umfangs (15) des absorbierenden Kerns (2, 9) verbindet,
wobei die flüssigkeitsundurchlässige Membran (6, 11) auf dem absorbierenden Kern (2, 9) platziert ist, wobei die Oberseite (16) des Kerns der flüssigkeitsundurchlässigen Membran (6, 11) zugewandt ist und der Umfang (15) des absorbierenden Kerns (2, 9) mit dem Umfang (21) der flüssigkeitsundurchlässigen Membran (6, 11) in Linie gebracht ist,
**dadurch gekennzeichnet, dass** die flüssigkeitsundurchlässige Membran (6, 11) mindestens die Abmessungen der Rückseite (17) des absorbierenden Kerns (2, 9) plus die Höhe (18) des Kerns plus den doppelten Abstand (19) der ersten Naht (20) zum Umfang (15) des absorbierenden Kerns (2, 9) aufweist, und wobei die flüssigkeitsundurchlässige Membran (6, 11) die Rückseite (17) des absorbierenden Kerns (2, 9), die Höhe (18) des absorbierenden Kerns (2, 9) und einen Randbereich, der dem Nähabstand (19) vom Umfang (15) des absorbierenden Kerns (2, 9) entspricht, umschließt.

10. Inkontinenzeinlage nach Anspruch 9, **dadurch gekennzeichnet, dass** sie mit einem Verfahren nach einem oder mehreren der Ansprüche 1-8 hergestellt wurde.

11. Verwendung einer Inkontinenzeinlage nach einem der Ansprüche 9 oder 10 in einer Unterhose.

12. Verwendung einer Inkontinenzeinlage nach einem der Ansprüche 9 oder 10 zusammen mit einem flüssigkeitsdichten Beutel, der versiegelt und zur Aufbewahrung des Produkts verwendet werden kann.

## Revendications

1. Procédé de production d'une garniture pour incontinence (1, 8) comprenant un coeur absorbant (2, 9), une membrane imperméable aux liquides (6, 11) et une première couture (20) reliant le coeur absorbant (2, 9) et la membrane imperméable aux liquides (6, 11), lequel procédé comprend au moins les étapes suivantes :
- la fourniture d'un coeur absorbant (2, 9) avec une circonférence (15) du coeur absorbant (2, 9), une face supérieure (16), une face arrière (17) et une hauteur du coeur (18), où la face supérieure (16) est orientée vers l'utilisateur et la face arrière est orientée à l'opposé de l'utilisateur lorsque ladite garniture pour incontinence (1, 8) est utilisée,
- la fourniture d'une membrane imperméable aux liquides (6, 11),
- la mise en place de ladite membrane imperméable aux liquides (6, 11) au-dessus dudit coeur absorbant (2, 9), avec la face supérieure (16) du coeur faisant face à ladite membrane imperméable aux liquides (6, 11), en alignant ladite circonférence (15) du coeur absorbant (2, 9) et ladite circonférence (21) de la membrane imperméable aux liquides (6, 11),
- la couture dudit coeur absorbant (2, 9) et de ladite membrane imperméable aux liquides (6, 11) ensemble le long d'une première partie (22) de la circonférence (15) du coeur absorbant (2, 9), laissant ainsi une seconde partie (23) de la circonférence (15) du coeur absorbant (19) non cousue, définissant un trou (24) entre le coeur absorbant relié (2, 9) et la membrane imperméable aux liquides (6, 11), et
- le retournement de l'intérieur du produit à travers le trou (24),
**caractérisé en ce que** ladite membrane imperméable aux liquides (6, 11) présente au moins les dimensions de la face arrière (17) dudit coeur absorbant (2, 9), plus la hauteur (18) du coeur, plus deux fois la distance (19) de ladite première couture (20) à ladite circonférence (15) du coeur absorbant (2, 9), et ladite première partie (22) de la circonférence (15) du coeur absorbant (2, 9) constitue une partie essentielle de la circonférence (15) du coeur absorbant (2, 9), de telle sorte qu'après avoir retourné l'intérieur du produit à travers le trou (24), la membrane imperméable aux liquides (6, 11) englobera la face arrière (17) du coeur absorbant (2, 9), la hauteur (18) dudit coeur absorbant (2, 9) et vers l'intérieur sur une zone de bord correspondant à la distance de couture (19) à partir de la circonférence (15) dudit coeur absorbant (2, 9), et dans lequel ledit procédé comprend également au moins les étapes suivantes :
- le pliage de la partie non cousue de la membrane imperméable aux liquides (6, 11) vers le haut le long de la hauteur (18) de et sur la région de bord du coeur absorbant (2, 9) dans ladite seconde partie (23) de la circonférence (15) du coeur absorbant (2, 9), et
- la couture de la partie non cousue de la membrane imperméable aux liquides (6, 11) au coeur absorbant (2, 9) dans ladite seconde partie (23) de la circonférence (15) du coeur absorbant (2, 9).

2. Procédé de production d'une garniture pour incontinence (1, 8) selon la revendication 1, **caractérisé en ce que** ledit procédé comprend également les étapes suivantes :
- la fourniture d'une couche supplémentaire de la membrane imperméable aux liquides, qui est repliée dans ladite seconde partie (23) de la circonférence (15) du coeur absorbant (2, 9) et la couture de la couche supplémentaire repliée de la membrane imperméable aux liquides à travers ladite seconde partie (23) de la circonférence (15) du coeur absorbant (2, 9), de sorte que la membrane imperméable aux liquides repliée soit cousue sur la face supérieure et la face arrière du coeur absorbant.

3. Procédé de production d'une garniture pour incontinence (1, 8) selon l'une quelconque des revendications précédentes, **caractérisé en ce que** ledit procédé comprend également les étapes suivantes :
- le pliage de la membrane imperméable aux liquides (6, 11) en plis (29) dans des zones situées à proximité des coins du coeur absorbant (2, 9) lorsque la circonférence (15) du coeur absorbant (2, 9) et la circonférence (21) de la membrane imperméable aux liquides (6, 11) sont alignées,
- la couture desdits plis (29).

4. Procédé de production d'une garniture pour incontinence (1, 8) selon l'une quelconque des revendications précédentes, **caractérisé en ce que** ledit procédé comprend également les étapes suivantes :
- la fourniture à ladite membrane imperméable aux liquides (6, 11) d'au moins deux boutons-pression étanches (7).

5. Procédé de production d'une garniture pour incontinence (1, 8) selon l'une quelconque des revendications précédentes, **caractérisé en ce que** ledit procédé comprend également les étapes suivantes :
- la fourniture dudit coeur absorbant (2, 9) sous une forme comprenant une couche de feutre absorbant l'eau (4) et au moins un tissu perméable à l'eau (3) disposé sur une face du feutre absorbant l'eau (4), et éventuellement un autre tissu perméable à l'eau (3) disposé sur l'autre face du feutre absorbant l'eau (4).

6. Procédé de production d'une garniture pour incontinence (1, 8) selon l'une quelconque des revendications précédentes, **caractérisé en ce que** ledit procédé comprend également les étapes suivantes :
- la fourniture dudit coeur absorbant (2, 9) comprenant la couture d'un, de deux ou de plus de tissus perméables à l'eau (3) .

7. Procédé de production d'une garniture pour incontinence (1, 8) selon l'une quelconque des revendications précédentes, **caractérisé en ce que** ledit procédé comprend également les étapes suivantes :
- la fourniture dudit coeur absorbant (2, 9) comprenant la couture d'au moins une première partie (25) du coeur absorbant (2, 9) et d'une seconde partie (26) du coeur absorbant (2, 9) ensemble,
- la couture d'une première partie (27) de la circonférence (21) de la membrane imperméable aux liquides (6, 11) et d'une seconde partie (28) de la circonférence (21) de la membrane imperméable aux liquides (6, 11) ensemble définissant une seconde couture (31), laquelle seconde couture façonne la membrane imperméable aux liquides en une forme qui peut s'adapter aux organes génitaux masculins.

8. Procédé de production d'une garniture pour incontinence (1, 8) selon la revendication 7, **caractérisé en ce que** ledit procédé comprend également les étapes suivantes :
- la fourniture d'une membrane imperméable aux liquides auxiliaire (30), qui est cousue au coeur absorbant (2, 9), dans une position où ladite membrane imperméable aux liquides auxiliaire (30) est placée entre le coeur absorbant (2, 9) et la membrane imperméable aux liquides (6, 11) dans le produit final, laquelle membrane imperméable aux liquides auxiliaire (30) chevauche une partie ou la totalité de la seconde couture (31).

9. Garniture pour incontinence comprenant un coeur absorbant (2, 9) avec une circonférence (15) du coeur absorbant (2, 9), une face supérieure (16), une face arrière (17) et une hauteur du coeur (18), où la face supérieure (16) est orientée vers l'utilisateur et la face arrière est orientée à l'opposé de l'utilisateur lorsque ladite garniture pour incontinence (1, 8) est utilisée, une membrane imperméable aux liquides (6, 11), et une première couture (20) reliant le coeur absorbant (2, 9) et la membrane imperméable aux liquides (6, 11) le long d'une première partie (22) de la circonférence (15) du coeur absorbant (2, 9), et une seconde couture reliant la membrane imperméable aux liquides (6, 11) au coeur absorbant (2, 9) dans une seconde partie (23) de la circonférence (15) du coeur absorbant (2, 9),
dans laquelle ladite membrane imperméable aux liquides (6, 11) est placée au-dessus dudit coeur absorbant (2, 9), avec la face supérieure (16) du coeur faisant face à ladite membrane imperméable aux liquides (6, 11), et ladite circonférence (15) du coeur absorbant (2, 9) étant alignée avec ladite circonférence (21) de la membrane imperméable aux liquides (6, 11),
**caractérisée en ce que** ladite membrane imperméable aux liquides (6, 11) présente au moins les dimensions de la face arrière (17) dudit coeur absorbant (2, 9), plus la hauteur (18) du coeur, plus deux fois la distance (19) de ladite première couture (20) à ladite circonférence (15) du coeur absorbant (2, 9), et dans laquelle ladite membrane imperméable aux liquides (6, 11) englobe la face arrière (17) du coeur absorbant (2, 9), la hauteur (18) dudit coeur absorbant (2, 9) et une zone de bord correspondant à la distance de couture (19) à partir de la circonférence (15) dudit coeur absorbant (2, 9).

10. Garniture pour incontinence selon la revendication 9, **caractérisée en ce qu'**elle a été produite par un procédé selon l'une quelconque ou plusieurs des revendications 1 à 8.

11. Utilisation d'une garniture pour incontinence selon l'une quelconque des revendications 9 ou 10 dans une paire de slips.

12. Utilisation d'une garniture pour incontinence selon l'une quelconque des revendications 9 ou 10 conjointement avec un sac étanche aux liquides, lequel sac peut être scellé et utilisé pour le stockage dudit produit.
